# EUROPÄISCHE PATENTSCHRIFT

(11) **EP 1 067 916 B1**
(45) Veröffentlichungstag und Bekanntmachung des Hinweises auf die Patenterteilung: **28.05.2003**
(21) Anmeldenummer: 99917831.2
(22) Anmeldetag: 20.03.1999
(51) Int. Cl.: A61K 9/70

(54) **VERFAHREN ZUR HERSTELLUNG VON TRANSDERMALEN THERAPEUTISCHEN SYSTEMEN UNTER VERWENDUNG VON BASISCHEN ALKALIMETALLSALZEN ZUR UMWANDLUNG VON WIRKSTOFFSALZEN IN DIE FREIEN BASEN**
METHOD FOR PRODUCING TRANSDERMAL THERAPEUTIC SYSTEMS BY USING BASIC ALKALI METAL SALTS FOR CONVERTING ACTIVE AGENT SALTS INTO FREE BASES
PROCEDE POUR PRODUIRE DES SYSTEMES THERAPEUTIQUES TRANSDERMIQUES AU MOYEN DE SELS DE METAUX ALCALINS BASIQUES SERVANT A TRANSFORMER DES SELS DE PRINCIPES ACTIFS EN BASES LIBRES

(30) Priorität: 30.03.1998 DE 19814083
(43) Veröffentlichungstag der Anmeldung: 17.01.2001
(73) Patentinhaber: LTS Lohmann Therapie-Systeme AG, 56626 Andernach (DE)
(72) Erfinder: MÜLLER, Walter, D-56564 Neuwied (DE)
(74) Vertreter: Flaccus, Rolf-Dieter, Dr.
(86) Internationale Anmeldenummer: EP9901876
(87) Internationale Veröffentlichungsnummer: WO99049844

(56) Entgegenhaltungen:
- EP-A- 0 593 807

## Beschreibung

Viele medizinische Wirkstoffe enthalten ein oder mehrere basische Stickstoffatome im Molekül und können deshalb entweder als freie Base oder als Salz der Wirkstoffbase mit einer für diesen Zweck geeigneten Säure in pharmazeutischen Zubereitungen eingesetzt werden. Salze haben den für die orale Verabreichung wichtigen Vorteil der besseren Wasserlöslichkeit und in vielen Fällen auch den der besseren St:abilität. Ein weiterer Vorteil liegt darin, daß Wirkstoffsalze oft leichter zu kristallisieren sind bzw. nur das Wirkstoffsalz bei Raumtemperatur überhaupt kristallin ist.
Dies ist der Grund, warum viele Wirkstoffe nur in Form ihrer Salze hergestellt und verfügbar sind.

Für die transdermale Verabreichung sind jedoch die Wirkstoffsalze ungeeignet, da sie aufgrund ihrer höheren Polarität die lipophile Barriere des Stratum Corneum nicht in der für den therapeutischen Zweck erforderlichen Menge durchdringen können.

Es ist deshalb erforderlich, Wirkstoffsalze für ihre Verwendung in transdermalen Systemen in ihre freie Base zu überführen.

Im wesentlichen dominieren zwei Typen von transdermalen therapeutischen Systemen (TTS) den Markt, nämlich die sogenannten Matrix- und Reservoirsysteme.

Ein Matrixsystem besteht im einfachsten Fall aus einer Rückschicht, einer selbstklebenden wirkstoffhaltigen Matrix und einer vor Gebrauch zu entfernenden Schutzfolie. In komplizierteren Ausführungen ist die Matrix mehrschichtig aufgebaut, wobei nicht jede Schicht auch selbstklebend sein muß. Auch der Einbau von Membranen in die Matrix zur Steuerung der Wirkstoffabgabe kann vorgesehen werden.

Ein Matrixsystem kann auch aus einer nichtklebenden wirkstoffhaitigen Matrix bestehen, die zur Befestigung auf der Haut mit einem diese Matrix an allen Seiten überragenden wirkstofffreien Überpflaster versehen ist.

Reservoirsysteme bestehen aus einem Beutel, der aus einer wirkstoffundurchlässigen Folie und einer zumindest für den Wirkstoff durchlässigen Membran gebildet wird. Der Beutel wird mit einer flüssigen oder gelförmigen Wirkstoffzubereitung gefüllt. Zur Verankerung auf der Haut ist die Membran in den meisten Fällen mit einem Kleber versehen. Auch diese Systeme sind mit einer vor Gebrauch zu entfernenden Schutzfolie versehen.

Technisch gesehen ist es natürlich kein Problem, ein Wirkstoffsalz in die freie Base zu überführen. Am einfachsten ist es, das Wirkstoffsalz in Wasser zu lösen und eine Hilfsbase wie z.B. NaOH zuzusetzen. Die entstehende Wirkstoffbase fällt aufgrund ihrer schlechteren Wasserlöslichkeit entweder aus und kann abfiltriert werden, oder sie muß mit einem geeigneten organischen Lösemittel, wie z.B. Methylenchlorid, extrahiert werden.
Nachteilig bei diesem Verfahren ist es, daß die freie Base speziell aufgearbeitet werden muß, damit sie für die Herstellung der transdermalen Systeme benutzt werden kann.

Ein ideales Verfahren erlaubt es, die freie Base während der Herstellung des Systems in situ freizusetzen, ohne daß sich das Verfahren dadurch wesentlich komplizierter als bei direkter Verwendung der freien Base gestaltet.

Ein solches Verfahren ist in der EP 0 272 562 beschrieben. Gemäß diesem Verfahren werden Kleber eingesetzt, die selbst über basische Gruppen verfügen und dadurch selbst als Hilfsbase zur Freisetzung der Wirkstoffbase fungieren könen. Der Nachteil dieses Verfahrens ist es, daß die Anzahl dieser funktionellen basischen Gruppen im Kleber beschränkt ist und dadurch nur geringe Mengen an Wirkstoffsalzen in ihre freien Basen überführt werden können. Ein weiteres Beispiel einer solchen in Situ-Umsetzung findet sich in der EP-A-0 593 807, in der die Freisetzung einer Wirkstoffbase - speziell von Deprenyl - mit Hilfe von Aminogruppen enthaltenden Polymeren beschrieben wird.

Aufgabe der vorliegenden Erfindung war es, ein Verfahren zu entwickeln, das auch die Umwandlung von größeren Wirkstoffmengen erlaubt, und demgemäß die Nachteile gemäß dem Stand der Technik vermeidet.

Überraschenderweise wurde gefunden, daß es möglich ist, Wirkstoffsalze in organischen Lösemitteln wie z.B. Methanol, Ethanol, Isopropanol, Methylethylketon durch Umsetzung mit basischen Alkalimetallsalzen, insbesondere -silikaten wie Trisilikaten und Metasilikaten des Natriums und Kaliums, in ihre freien Basen zu überführen. Trisilikate und Metasilikate sind in verschiedenen Hydratationsstufen erhältlich, die jedoch bezüglich ihrer Eignung als gleichwertig anzusehen sind.
Diese Silikate sind Salze einer schwachen Säure mit einer starken Base und reagieren deshalb basisch. Das heißt, daß sie in Anwesenheit von Wirkstoffsalzen, die als schwache Säuren zu betrachten sind, in die freien Kieselsäuren überführt werden. Die freien Kieselsäuren sind instabil und regieren unter Wasserabspaltung weiter zu polymerem Siliziumdioxid. Dadurch ist die Reaktion irreversibel und die Umwandlung der Wirkstoffsalze in ihre freien Basen ist trotz der geringen Basizität der Silikate vollständig möglich. Voraussetzung ist natürlich, daß zumindest die stöchiometrisch notwendige Menge an Silikat verwendet wird. Die Tatsache, daß die Reaktion irreversibel ist, macht Silikate anderen Hilfsbasen wie z.B. Ethanolaminen überlegen, da letztere Verbindungen über eine den Wirkstoffbasen vergleichbare Basizität verfügen und es deshalb lediglich zu Gleichgewichten kommt, bei denen eine nahezu quantitative Umwandlung des Wirkstoffsalzes in die freie Base nur unter Verwendung eines Überschusses an Hilfsbase möglich ist. Zudem können diese Hilfsbasen selbst in die Haut eindringen bzw. durch die Haut permeieren und zu lokalen Hautreizungen bzw. toxischen Nebenwirkungen führen.
Es ist überraschend und unerwartet, daß Alkalimetallsilikate, insbesondere Tri- und Metasilikate des Natriums und Kaliums, für diesen Zweck in organischen Lösemitteln eingesetzt werden können, da sowohl die Wirkstoffsalze als auch die Silikate in diesen Lösemitteln nur über eine sehr geringe Löslichkeit verfügen.
Die beste Löslichkeit für diese Silikate wurden in Methanol und Ethanol gefunden und zu lediglich 0,01 % (g/g) bestimmt. Trotzdem können sogar Lösemittel mit noch geringerer Löslichkeit für Silikate wie z.B. Isopropanol, Aceton, Methylethylketon, Ethylacetat und Mischungen der genannten Lösemittel verwendet werden.
Überraschend ist es zudem, daß es trotz dieser geringen Löslichkeit gelingt, in akzeptablen Zeiten eine komplette Umwandlung der Wirkstoffsalze in ihre freien Basen zu erreichen. Normalerweise dauert die komplette Umwandlung bei Raumtemperatur lediglich ca. 2-3 Tage; sie kann durch Temperaturerhöhung auf ca. 35-40 °C auf ca. 24 Stunden verkürzt werden. Versuche, Silikate von Calcium oder Magnesium einzusetzen, scheiterten, da sie, bedingt durch die mehrwertigen Kationen, in organischen Lösemitteln praktisch unlöslich sind. Als ebenso unbrauchbar haben sich basische aluminiumhaltige Mischsilikate erwiesen.

Die Vollständigkeit der Umwandlung kann mikroskopisch kontrolliert werden. Ist die Umwandlung abgelaufen, lassen sich keine der in organischen Lösemitteln schwerlöslichen Kristalle der Wirkstoffsalze mehr erkennen.

Für die Herstellung von Matrixsystemen ist wichtig, daß die Lösemittel, die zur Umsetzung verwendet werden, gut verträglich mit den in organischen Lösemitteln gelösten Klebern sind.
Dies ist bei den oben genannten Lösemitteln der Fall, jedoch ist die genannte Auswahl nur beispielhaft.

Reaktionsprodukte der Alkalisilikate sind Siliziumdioxid und das Natrium- bzw. Kaliumsalz der im Wirkstoff enthaltenen Säure. Siliziumdioxid ist eine Verbindung, die als absolut untoxisch zu betrachten ist. Es ist deshalb aus toxikologischer Sicht nicht nötig, Siliziumdioxid aus der WIrkstofflösung zu entfernen. Sollte es aus technischer Sicht notwendig sein, ist lediglich ein Filtrationsschritt vorzusehen.

Auch die basischen Silikate selbst sind als praktisch untoxisch anzusehen. Sie werden problemlos in vielen Industrieoder Haushaltreinigern eingesetzt, um den pH-Wert des Reinigers basisch zu stellen. Die einzige Reaktion, die möglicherweise zu erwarten ist, ist eine Hautreizung aufgrund ihrer Basizität. Da jedoch ihre Löslichkeit in den für Matrixsysteme verwendeten Polymeren bzw. Reservoirformulierungen gering ist, ist auch dies normalerweise nicht zu befürchten. Erst bei sehr großen Konzentrationen, die dazu führen, daß in Matrixsystemen die ungelösten Silikatkristalle in Kontakt mit der Haut kommen, sind lokale Hautreizungen zu befürchten. Durch Filtration ist allerdings ein Überschuß an Silikaten in der umgesetzten Wirkstofflösung sehr leicht zu entfernen. Der Filtrationsschritt empfiehlt sich, auch wenn der Wirkstoff in Gegenwart von basischen Substanzen zu Instabilitäten neigt. Nach Filtration ist der pH-Wert in der TTS-Matrix bzw. im Reservoir von Reservoirsystemen - wenn keine weiteren pH-Regulatoren zugesetzt wurden - nur durch die Basizität des Wirkstoffs selbst bestimmt.

Die Verwendung von basischen Alkalimetallsalzen, insbesondere Alkalimetallsilikaten, und speziell Meta- und Trisilikaten des Natrium bzw. Kaliums zur in-situ Umwandlung von Salzen basischer Wirkstoffe in die freien Wirkstoffbasen während der Herstellung von transdermalen therapeutischen Systemen stellt eine erhebliche Verbesserung gegenüber dem Stand der Technik dar. Die Umwandlung geschieht unter sehr milden Bedingungen und es ist nicht notwendig, die Wirkstoffbase zu isolieren bzw. die Reaktionsprodukte der Hilfsbase abzutrennen. Eventuell unverbrauchte Überschußmengen der Silikate brauchen ebenfalls nicht angetrennt zu werden, da infolge der Einarbeitung in das transdermale System keinerlei Nebenwirkungen zu befürchten sind.

### Beispiele:

### Beispiel 1:

20g (-)-5,6,7,8-Tetrahydro-6-[propyl[2-(2-thienyl)ethyl] amino]-1-naphthol-hydrochlorid werden zusammen mit 8,0 g Natriummetasilikat oder 9,1 g Natriumtrisilikat in 35 ml Ethanol über 48 Stunden bei Raumtemperatur gerührt. Optional wird die Wirkstofflösung nun filtriert und 6,0 g Polyvinylpyrrolidon (Kollidon F90, Fa. Bayer) in Form einer 25%igen (g/g) Lösung in Ethanol und 250 g einer 70%igen Lösung eines aminresistenten Silikonklebers (Q7-4301, Fa. Dow Corning) in Heptan zugegeben und die Masse anschließend durch mechanisches Rühren homogenisiert.

Anschließend wird die Masse zur Herstellung der Pflastermatrix auf eine geeignete, abhäsiv ausgerüstete Folie beschichtet und die Lösemittel durch 20 minütiges Trocknen bei 50 °C entfernt. Das Beschichtungsgewicht des getrockneten Matrixfilms liegt bei 50 g/m².

Der getrocknete Matrixfilm wird mit einer 23 µm dicken Polyesterfolie kaschiert. Die einzelnen Pflaster werden aus dem Gesamtlaminat gestanzt.

### Beispiel 2:

25g (-)-5,6,7,8-Tetrahydro-6-[propyl[2-(2-thienyl)ethyl] amino]-1-naphthol-hydrochlorid werden zusammen mit 14,7 g Natriummetasilikat oder 16,8 g Natriumtrisilikat in 40 ml Ethanol über 48 Stunden bei Raumtemperatur gerührt. Optional wird die Wirkstofflösung nun filtriert und 9,2 g Oleylalkohol, 63,2 g einer 52 %igen Lösung eines Polyacrylatklebers (Durotak 387-2287, Fa. National Starch & Chemical) und 22,8 g einer 40 % (g/g) Lösung von Eudragit E 100 (Röhm-Pharma) zugegeben und die Masse anschließend durch mechanisches Rühren homogenisiert.

Anschließend wird die Masse zur Herstellung der Pflastermatrix auf eine geeignete, abhäsiv ausgerüstete Folie beschichtet und die Lösemittel durch 20 minütiges Trocknen bei 50 °C entfernt. Das Beschichtungsgewicht des getrockneten Matrixfilms liegt bei 80 g/m².

Der getrocknete Matrixfilm wird mit einer 23 µm dicken Polyesterfolie kaschiert. Die einzelnen Pflaster werden aus dem Gesamtlaminat gestanzt.

### Beispiel 3:

50 g Scopolaminhydrobromid werden zusammen mit 13,8 g Natriummetasilikat oder 15,7 g Natriumtrisilikat in 40 ml Ethanol über 48 Stunden bei Raumtemperatur gerührt. Optional wird die Wirkstofflösung nunfiltriert und 32 g Ölsäure und 480 g einer 52 %igen Lösung eines Polyacrylatklebers (Durotak 387-2253, Fa. National Starch & Chemical) zugegeben und die Masse anschließend durch mechanisches Rühren homogenisiert.

Anschließend wird die Masse zur Herstellung der Pflastermatrix auf eine geeignete, abhäsiv ausgerüstete Folie beschichtet und die Lösemittel durch 20 minütiges Trocknen bei 50 °C entfernt. Das Beschichtungsgewicht des getrockneten Matrixfilms liegt bei 90 g/m².

Der getrocknete Matrixfilm wird mit einer 23 um dicken Polyesterfolie kaschiert. Die einzelnen Pflaster werden aus dem Gesamtlaminat gestanzt.

## Patentansprüche

1. Verfahren zur Herstellung von transdermalen Systemen mit freien Wirkstoffbasen, **dadurch gekennzeichnet, daß** die freie Wirkstoffbase während der Herstellung des Systems aus Wirkstoffsalzen durch Umsetzung mit einem basischen Alkalimetallsalz freigesetzt wird.

2. Verfahren zur Herstellung von transdermalen Systemen mit freien Wirkstoffbasen nach Anspruch 1, **dadurch gekennzeichnet, daß** das basische Alkalimetallsalz ein Silikat ist.

3. Verfahren zur Herstellung von transdermalen Systemen mit freien Wirkstoffbasen nach Anspruch 2, **dadurch gekennzeichnet, daß** das Silikat ein Natrium- oder Kaliumsilikat ist.

4. Verfahren zur Herstellung von transdermalen Systemen mit freien Wirkstoffbasen nach Anspruch 2 oder 3, **dadurch gekennzeichnet, daß** das Silikat ein Tri- oder Metasilikat ist.

5. Verfahren zur Herstellung von transdermalen Systemen mit freien Wirkstoffbasen nach einem oder mehreren der vorangehenden Ansprüche, **dadurch gekennzeichnet, daß** die Umsetzung in einem organischen Lösemittel erfolgt.

6. Verfahren zur Herstellung von transdermalen Systemen mit freien Wirkstoffbasen nach Anspruch 5, **dadurch gekennzeichnet, daß** das organische Lösemittel aus Ethanol, Methanol, Methylethylketon, Isopropanol, Ethylenglykol, Propylenglykol oder deren Mischungen besteht.

7. Verfahren zur Herstellung transdermaler Systeme nach Anspruch 5, **dadurch gekennzeichnet, daß** das Wirkstoffsalz mit dem basischen Alkalimetallsalz in dem organischen Lösemittel suspendiert wird, die Suspension bis zur quantitativen Umsetzung des Wirkstoffsalzes gerührt wird und anschließend zur Herstellung eines transdermalen oder topischen Systems einer in einem organischen Lösemittel gelösten Polymermasse zugesetzt wird.

8. Verfahren zur Herstellung von transdermalen Systemen nach Anspruch 7, **dadurch gekennzeichnet, daß** die gelöste Polymermasse einen Kleber darstellt.

9. Verfahren zur Herstellung von transdermalen Systemen nach Anspruch 7, **dadurch gekennzeichnet, daß** die Lösung bzw. Suspension des basischen Alkalimetallsalzes und des Wirkstoffsalzes nach Umsetzung und vor Zugabe zur Polymermasse filtriert wird.

10. Verfahren zur Herstellung eines transdermalen Systems nach Anspruch 8 oder 9, **dadurch gekennzeichnet, daß** die Polymermasse zur Herstellung eines Matrixsstems auf eine geeignete, adhäsiv ausgerüstete Folie beschichtet wird, die Lösemittel durch Trocknen entfernt werden, der getrocknete Film mit einer geeigneten Folie kaschiert wird und aus dem entstandenen Laminat die transdermalen Systeme ausgestanzt werden.

11. Verfahren zur Herstellung eines transdermalen Systems nach Anspruch 8 oder 9, **dadurch gekennzeichnet, daß** die Lösung nach erfolgter Umsetzung des Wirkstoffsalzes und gegebenenfalls dem Zusatz von weiteren Hilfsstoffen zur Herstellung eines Reservoirsystems in einen Beutel, bestehend aus einer undurchlässigen Rückschicht und einer zumindest für den Wirkstoff durchlässigen Membran, gefüllt wird.

12. Verfahren zur Herstellung eines transdermalen Systems nach Anspruch 11, **dadurch gekennzeichnet, daß** die Membran des Beutels mit einer Kleberschicht zur Haftung auf der Haut versehen ist.

13. Verfahren zur Herstellung von transdermalen Systemen mit Wirkstoff nach Anspruch 1, **dadurch gekennzeichnet, daß** der Wirkstoff zur Gruppe der Antihypertonika, Antiemitika, Anti-Parkinsonmittel, Antidepressiva, Antiasthmatika, Analgetika oder Antiallergika gehört.

14. Verfahren zur Herstellung eines transdermalen Systems nach Anspruch 1, **dadurch gekennzeichnet, daß** der Wirkstoff ein D2-Agonist und speziell (-)-5,6,7,8-Tetrahydro-6-[propyl[2-(2-thienyl)ethyl]amino]-1-naphthol ist.

## Claims

1. A process for manufacturing transdermal systems comprising free active substance bases, **characterized in that** the free active substance base is liberated, during the manufacture of the system, from active substance salts by conversion with a basic alkaline metal salt.

2. The process for manufacturing transdermal systems comprising free active substance bases according to Claim 1, **characterized in that** the basic alkaline metal salt is a silicate.

3. The process for manufacturing transdermal systems comprising free active substance bases according to Claim 2, **characterized in that** the silicate is a sodium silicate or potassium silicate.

4. The process for manufacturing transdermal systems comprising free active substance bases according to Claim 2 or 3, **characterized in that** the silicate is a trisilicate or metasilicate.

5. The process for manufacturing transdermal systems comprising free active substance bases according to one or more of the preceding claims, **characterized in that** the conversion takes place in an organic solvent.

6. The process for manufacturing transdermal systems comprising free active substance bases according to Claim 5, **characterized in that** the organic solvent consists of ethanol, methanol, methyl ethyl ketone, isopropanol, ethylene glycol, propylene glycol or mixtures thereof.

7. The process for manufacturing transdermal systems according to Claim 5, **characterized in that** the active substance salt is suspended along with the basic alkaline metal salt in the organic solvent, the suspension is stirred until quantitative conversion of the active substance salt, and, for the manufacture of a transdermal or topical system, is subsequently added to a polymer mass which is dissolved in an organic solvent.

8. The process for manufacturing transdermal systems according to Claim 7, **characterized in that** the dissolved polymer mass is an adhesive.

9. The process for manufacturing transdermal systems according to Claim 7, **characterized in that** the solution or suspension of the basic alkaline metal salt and the active substance salt is filtered following the conversion and prior to being added to the polymer mass.

10. The process for manufacturing transdermal systems according to Claim 8 or 9, **characterized in that**, for manufacture of a matrix system, the polymer mass is coated on a suitable, adhesively equipped film or sheet, the solvents are removed by drying, the dried film is laminated with a suitable film or sheet, and the transdermal systems are punched out from the resultant laminate.

11. The process for manufacturing transdermal systems according to Claim 8 or 9, **characterized in that**, for manufacture of a reservoir system, the solvent is filled, after complete conversion of the active substance salt and possible addition of further auxiliary substances, into a bag consisting of an impermeable backing layer and a membrane which is permeable at least to the active substance.

12. The process for manufacturing transdermal systems according to Claim 11, **characterized in that** the membrane of the bag is provided with an adhesive layer for adhesion to the skin.

13. The process for manufacturing transdermal systems according to Claim 1, **characterized in that** the active substance belongs to the group of antihypertensives, antiemetics, antiparkinsonian agents, antidepressants, antiasthmatics, analgesics or antiallergic agents.

14. The process for manufacturing transdermal systems according to Claim 1, **characterized in that** the active substance is a D2-agonist and especially (-)-5,6,7,8-tetrahydro-6-[propyl[2-(2-thienyl)ethyl]amino]-1-naphthol.

## Revendications

1. Procédé pour produire des systèmes transdermiques avec des bases libres de principe actif, **caractérisé en ce que** la base libre de principe actif est libérée pendant la production du système à partir de sels de principe actif, par réaction avec un sel basique de métal alcalin.

2. Procédé pour produire des systèmes transdermiques avec des bases libres de principe actif selon la revendication 1, **caractérisé en ce que** le sel basique de métal alcalin est un silicate.

3. Procédé pour produire des systèmes transdermiques avec des bases libres de principe actif selon la revendication 2, **caractérisé en ce que** le silicate est un silicate de sodium ou un silicate de potassium.

4. Procédé pour produire des systèmes transdermiques avec des bases libres de principe actif selon la revendication 2 ou 3, **caractérisé en ce que** le silicate est un trisilicate ou métasilicate.

5. Procédé pour produire des systèmes transdermiques avec des bases libres de principe actif selon l'une ou plusieurs des revendications précédentes, **caractérisé en ce que** la conversion se produit dans un solvant organique.

6. Procédé pour produire des systèmes transdermiques avec des bases libres de principe actif selon la revendication 5, **caractérisé en ce que** le solvant organique est constitué d'éthanol, de méthanol, de méthyléthylcétone, d'isopropanol, d'éthylèneglycol, de propylèneglycol, ou de leurs mélanges.

7. Procédé pour produire des systèmes transdermiques selon la revendication 5, **caractérisé en ce que** le sel de principe actif avec le sel basique de métal alcalin, est mis en suspension dans le solvant organique, la suspension est agitée jusqu'à conversion quantitative du sel de principe actif, puis ajoutée à une masse polymère dissoute dans un solvant organique pour produire un système transdermique ou topique.

8. Procédé pour produire des systèmes transdermiques selon la revendication 7, **caractérisé en ce que** la masse polymère dissoute représente une colle.

9. Procédé pour produire des systèmes transdermiques selon la revendication 7, **caractérisé en ce que** la solution ou la suspension du sel basique de métal alcalin et du sel de principe actif, sont filtrées après la conversion et avant l'ajout à la masse polymère.

10. Procédé pour produire un système transdermique selon la revendication 8 ou 9, **caractérisé en ce que** pour produire un système de matrice, la masse polymère est déposée sur une feuille appropriée, apprêtée de façon à adhérer, les solvants sont éliminés par séchage, le film séché est contrecollé avec une feuille appropriée, et les systèmes transdermiques sont découpés à la matrice à partir du stratifié formé.

11. Procédé pour produire un système transdermique selon la revendication 8 ou 9, **caractérisé en ce que** la solution, après une conversion réussie du sel de principe actif et éventuellement l'ajout d'autres additifs pour produire un système à réservoir, est introduite dans un sachet, constitué d'une couche support imperméable et d'une membrane au moins perméable au principe actif.

12. Procédé pour produire un système transdermique selon la revendication 11, **caractérisé en ce que** la membrane du sachet est munie d'une couche de colle pour adhérer à la peau.

13. Procédé pour produire des systèmes transdermiques avec principe actif selon la revendication 1, **caractérisé en ce que** le principe actif appartient au groupe des antihypertoniques, antiémétiques, antiparkinsoniens, antidépresseurs, antiasthmatiques, analgésiques ou antiallergiques.

14. Procédé pour produire un système transdermique selon la revendication 1, **caractérisé en ce que** le principe actif est un agoniste D2 et en particulier le (-)-5,6,7,8-tétrahydro-6-[propyl[2-(2-thiényl)éthyl]amino]-1-naphtol.
